# EUROPÄISCHE PATENTSCHRIFT

(11) **EP 0 686 036 B1**
(45) Veröffentlichungstag und Bekanntmachung des Hinweises auf die Patenterteilung: **12.03.1997**
(21) Anmeldenummer: 93917546.9
(22) Anmeldetag: 09.08.1993
(51) Int. Cl.: A61K 31/557

(54) **VERWENDUNG VON PROSTANDERIVATEN ZUR HERSTELLUNG EINES ARZNEIMITTELS ZUR BEHANDLUNG DER CHRONISCHEN POLYARTHRITIS**
USE OF PROSTANE DERIVATIVES OF FORMULAE I AND II FOR THE PRODUCTION OF A MEDICAMENT FOR THE TREATMENT OF CHRONIC POLYARTHRITIS
UTILISATION DE DERIVES DE PROSTANE DE FORMULES (I) ET (II) POUR LA PRODUCTION D'UN MEDICAMENT DESTINE AU TRAITEMENT DE LA POLYARTHRITE CHRONIQUE

(30) Priorität: 07.08.1992 DE 4226615; 19.08.1992 DE 4227788
(43) Veröffentlichungstag der Anmeldung: 13.12.1995
(73) Patentinhaber: SCHERING AKTIENGESELLSCHAFT, 13353 Berlin (DE)
(72) Erfinder: SCHOLZ, Peter, D-12209 Berlin (DE)
(86) Internationale Anmeldenummer: DE9300722
(87) Internationale Veröffentlichungsnummer: WO9403175

(56) Entgegenhaltungen:
- EP-A- 0 011 591
- EP-A- 0 084 856
- BR. J. PLAST. SURG., Bd. 37, Nr. 2, 1984, Seiten 175-178; S.KAY ET AL.: 'Spontaneous healing and relief of pain in a patient with intractable vasculitic ulceration of the lower limb following an intravenous infusion of prostacyclin: a case report', Zusammenfassung, S. 178
- ARTHRITIS AND RHEUMATISM, Bd. 24, Nr. 9, 1981, Seiten 1151-1158; STEVEN L. KUNKEL ET AL.: 'Suppression of acute and chronic inflammation by orally administered prostaglandins', Zusammenfassung, S. 1152

## Beschreibung

Die Erfindung betrifft die Verwendung von Prostan-Derivaten zur Herstellung eines Medikaments Zur Behandlung einer Immunantwort.

In der Publikation EP 0 011 591 (Anmeldetag: 18. Oktober 1979) werden einige Prostan-Derivate und deren Herstellung beschrieben. Bei diesen Prostan-Derivaten handelt es sich um Verbindungen, die vom Prostacyclin (PGl₂) abgeleitet sind. Sie enthalten eine Methylengruppe an Stelle des 9-Ether-Sauerstoff-Atoms im Prostacyclin. Prostan-Derivate werden zur Behandlung verschiedener Krankheiten eingesetzt, wobei die cardiovaskuläre und thromboaggregationshemmende Wirkung klar im Vordergund steht.

Die Verwendung von Prostan-Derivaten als Medikament wird ausführlich in der euopäischen Publikation EP 0 011 591 beschreiben:
Senkung des peripheren, arteriellen und koronaren vaskulären Widerstandes, Inhibierung der Thrombozytenaggregation und Auflösung von Plättchenthromben, myocardiale Zytoprotektion und damit Senkung des systemischen Blutdruckes ohne zugleich Schlagvolumen und koronare Durchblutung zu senken; Behandlung von Schlaganfall, Prophyiaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion und Zytoprotektion der Magen- und Darmschleimhaut; antiallergische Eigenschaften, Senkung des pulmonaren vaskulären Widerstandes und des pulmonaren Blutdruckes, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, antiproliferative Wirkung, Erhöhung der cerebralen Durchblutung.

In den Publikationen EP-0 055 208, EP-0 099 538 und EP-0 119 949 werden Carbacyclin-Derivate aufgeführt, die ähnliche Indikationen wie die zuvor erwähnten Prostan-Derivate besitzen.

Weitere Prostan-Derivate sind in der Publikation EP 0 084 856 beschrieben, die für die Verwendung bei der Inhibierung der Thrombozyten-Aggregation, Senkung des systemischen Blutdrucks oder der Behandlung von Magengeschwüren vorgeschlagen wurden. Darin ist insbesondere Beraprost erwähnt.

Die Verwendung von Prostan-Derivaten zur Behandlung von Immunantworten wird in verschiedenen Publikationen beschrieben. So wird die Behandlung von antiallergischen Eigenschaften schon in der europäischen Publikation EP 0 011 591 beiläufig erwähnt.

Die euopäischen Publikationen EP 0 055 208 beschreibt unter anderem die antiallergische Wirkung von Carbacyclin-Derivaten.

In der Publikation von H.J. GRUNDMANN et al. (1992) J. Infect. Dis. 1992, **165**: 1-5 wird die Verwendung von einem Prostan-Derivat, nämlich Iloprost, bei der Behandlung von septischem Schock ausführlich dargelegt.

In der internationalen Patentanmeldung PCT/DE 92/00100 (Anmeldetag der prioritätsbegründenden Anmeldung: 12. Februar 1991 in der Bundesrepublik Deutschland) wird die Therapie von AIDS und Diabetes mit Hilfe von Prostan-Derivaten erwähnt. Diese Anmeldung ist älteres Recht.

Die Publikation K. SLIWA et al. (1991) Infection and Immunity, **59**: 3846-3848, befaßt sich mit der Behandlung von zerebraler Malaria mit Iloprost.

Es wurde nun überraschenderweise gefunden, daß Prostan-Derivate bei einer weiteren Indikation eingesetzt werden können.

Die Erfindung betrifft eine Verwendung von Prostan-Derivaten der allgemeinen Formel I oder II 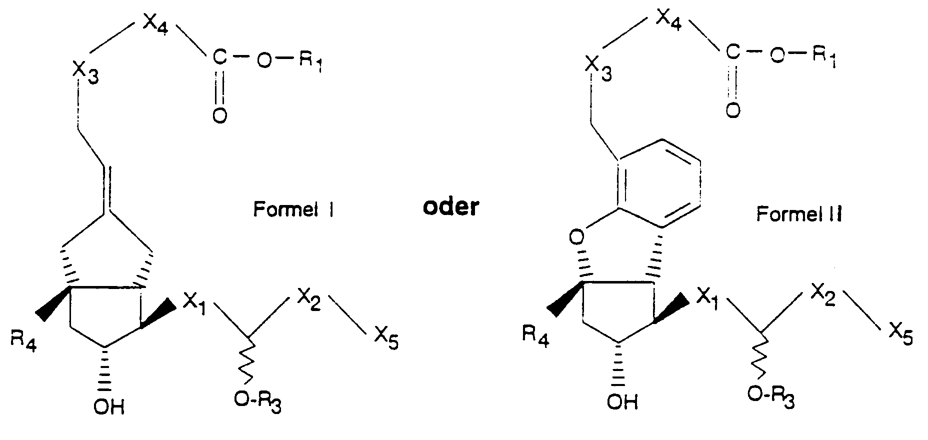 worin
- X₁: ein -CH₂-CH₂-; trans -CH=CH- oder -C≡C- ist,
- X₂: eine geradkettige oder verzweigte gesättigte Alkylengruppe mit 1 bis 6 Kohlenstoff-Atomen ist,
- X₃: ein -O- oder -CH₂- ist,
- X₄: ein -CH₂- oder -[CH₂]₃- ist,
- X₅: ein -H oder -C≡C-R₂ ist;
- R₁: ein Wasserstoff-Atom, eine Alkylgruppe mit 1 bis 6 Kohlenstoff-Atomen, eine Cycloalkylgruppe mit 5 oder 6 Kohlenstoff-Atomen oder Phenylgruppe ist,
- R₂: eine gerad- oder verzweigtkettige, gesättigte oder ungesättigte Alkylgruppe mit 1 bis 6 Kohlenstoff-Atomen ist,
- R₃: ein Wasserstoff-Atom, ein Acylrest mit 1 bis 4 Kohlenstoff-Atomen oder ein Benzoylrest ist und
- R₄: ein -H oder -CH₃ ist;
wobei die -O-R₃-Gruppe α- oder β- ständig ist, und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

X₂ steht für geradkettige oder verzweigte gesättigte Alkylengruppen mit 1 bis 6 Kohlenstoff-Atomen, so beispielsweise Methylen, Ethylen, Propylen, Isopropylen, wobei die Methylgruppe mit dem ersten oder zweiten Kohlenstoff-Atom des Ethylen, gerechnet von der Gruppe A, verbunden ist; Butylen, Methylpropylen, Ethylethylen, Dimethylethylen, wobei die Methyl- oder Ethylgruppen beliebig mit der Alkylenkette verbunden ist; Pentyl, Methylbutylen, Dimethylpropylen, Ethylpropylen, Methylethylethylen, wobei die Methyl- oder Ethylgruppen beliebig mit der Alkylenkette verbunden ist; Hexylen, Methylpentylen, Dimethylbutylen, Methylethylpropylen, wobei die Methyl- oder Ethylgruppe beliebig mit der Alkylenkette verbunden ist.

Die Alkylgruppen R₁ umfaßt gerade oder verzweigte Alkylgruppen mit 1 bis 6 Kohlenstoff-Atomen, wie beispielsweise Methyl, Ethyl, Propyl, Butyl, Isobutyl, tert.-Butyl, Pentyl, Neopentyl, Hexyl. Die Cycloalkylgruppe R₁ kann im Ring 5 oder 6 Kohlenstoff-Atome enthalten.

Die Alkylgruppe R₂ kann aus gerad- oder verzweigtkettigen, gesättigten oder ungesättigten Alkylresten mit 1 bis 6 Kohlenstoff-Atomen bestehen, vorzugsweise sind die Alkylreste gesättig. Beispielsweise genannt seien Methyl-, Ethyl-, Propyl-, Butyl-, Isobutyl-, tert.-Butyl-, Pentyl-, Hexyl-, Butenyl-, Isobutenyl-, Propenyl-, Pentenyl- oder Hexenyl-Gruppen.

Die Acylgruppe R₃ kann aus einer gerad- oder verzweigtkettigen Acylgruppe mit 1 bis 4 Kohlenstoff-Atomen bestehen, wie beispielsweise Acetyl, Propionyl, Butyryl oder Isobutyryl.

Die Erfindung betrifft ebenfalls ein Verfahren zur Behandlung von chronischer Polyarthritis bei Menschen und Säugetieren, die eine solche Behandlung benötigen, wobei die Behandlung eine Verabreichung einer pharmakologisch sicheren und wirkungsvollen Menge der Prostan-Derivate gemäß der Formel I oder II bei Menschen und Säugetieren umfaßt.

Zur Salzbildung mit den freien Säuren sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w. Die β-Cyclodextrinclathrat-Bildung erfolgt entsprechend EP 0 259 468.

Die chronische Polyarthritis ist eine Erkrankung der Gelenke, wobei das Synovialgewebe einen destruktiven Effekt auf die Matrix des Gelenkknorpels ausübt. In dem Synovialgewebe und der Synovialflüssigkeit von Patienten mit chronischer Polyarthritis befinden sich aktivierte Zellen, die Zytokine wie TNF-α und II-1 sekretieren. Beide Zytokine, TNF-α und II-1, sind in der Lage, die Produktion von Kollagenase und anderen neutralen Proteasen in synovialen Fibroblasten und Chondrozyten zu induzieren, die sich in der Nähe des Gelenkknorpels befinden. Diese Enzyme bauen Proteoglykane und Kollagen ab, was zu einer Zerstörung des Knorpels führt. (TNF-α = Tumor Necrosis Factor-α; II-1 = Interlenkin 1)

Überraschenderweise eignen sich Prostan-Derivate zur Behandlung der chronischen Polyarthritis. Bisher wurden zur Therapie die Substanzen wie Carboxylsäuren (Salizylate, Arylessigsäuren, Fenamate, Propionsäuren) Pyrazolone und Oxicame verwendet. (Handbook of Experimental Pharmacoly, 1979, Springer Verlag, Vol. **50**/**II**, Editors: G.V.R. BORN et al., Chapter 30, Anti-Inflammatory Drugs, J.R. VANE, S.H. FERREIRA, Chapter 32, Classification of Antirheumatic Drugs, E.C. HUSKISSON, Chapter 37, M.K. JASANI)

Bevorzugt ist die Verwendung von erfindungsgemäßen Prostan-Derivaten mit der zuvor genannten allgemeinen Formel I
worin
- X₁: ein trans -CH=CH- ist,
- X₂: eine geradkettige oder verzweigte gesättigte Alkylengruppe mit 2 bis 4 Kohlenstoff-Atomen ist,
- X₃: ein -CH₂- ist,
- X₄: ein -CH₂- ist,
- X₅: ein -C≡C-R₂ ist;
- R₁: ein Wasserstoff-Atom, eine Alkylgruppe mit 1 bis 3 Kohlenstoff-Atomen oder Phenylgruppe ist,
- R₂: eine gerad- oder verzweigtkettige, gesättigte oder ungesättigte Alkylgruppe mit 1 bis 3 Kohlenstoff-Atomen ist,
- R₃: ein Wasserstoff-Atom oder ein Acylrest mit 2 Kohlenstoff-Atomen ist und
- R₄: ein -H ist;
wobei die -O-R₃-Gruppe α- oder β- ständig ist,
und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

Mehr bevorzugt ist die Verwendung von erfindungsgemäßen Prostan-Derivaten mit der zuvor genannten allgemeinen Formel I
worin
- X₁: ein trans -CH=CH- ist,
- X₂: eine Methylethylengruppe ist, wobei die Methylgruppe mit dem ersten Kohlenstoff-Atom der Ethylengruppe verbunden ist und das erste Kohlenstoff-Atom der Ethylengruppe zu der Gruppe A weist,
- X₃: ein -CH₂- ist,
- X₄: ein -CH₂- ist,
- X₅: ein -C≡C-R₂ ist;
- R₁: ein Wasserstoff-Atom oder eine Methylgruppe ist,
- R₂: eine Methyl- oder Ethylgruppe ist,
- R₃: ein Wasserstoff-Atom oder eine Formylgruppe ist und
- R₄: ein -H ist;
wobei die -O-R₃-Gruppe α- oder β- ständig ist, und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

Am meisten bevorzugt ist die Verwendung von einem erfindungsgemäßen Prostan-Derivat mit der zuvor genannten allgemeinen Formel I
worin
- X₁: trans -CH=CH- ist,
- X₂: eine Methylethylengruppe ist, wobei die Methylgruppe mit dem ersten Kohlenstoff-Atom der Ethylengruppe verbunden ist und das erste Kohlenstoff-Atom der Ethylengruppe zu der Gruppe A weist,
- X₃: ein -CH₂- ist,
- X₄: ein -CH₂- ist
- X₅: ein -C≡C-R₂ ist;
- R₁: ein Wasserstoff-Atom ist,
- R₂: eine Methlygruppe ist,
- R₃: ein Wasserstoff-Atom ist und
- R₄: ein -H ist;
wobei die OH-Gruppe α- oder β- ständig ist,
und dessen Salze mit physiologisch verträglichen Basen zur Herstellung eines Arzneimittels zur Behandlung von chronischer Polyarthritis.

Diese Substanz besitzt den Namen "Iloprost" und trägt die systematische Bezeichnung 5-(E)-(1S,5S,6R)-7-Hydroxy-6[(E)-(3S,4RS)-3-hydroxy-4-methyl-1-octen-6-inyl]bicyclo[3.3.0]octen-3-yliden pentansäure. Sie ist die am meisten bevorzugte Verbindung dieser Erfindung.

Weiterhin umfaßt die Erfindung die Verwendung der bevorzugten Substanzen Cicaprost, Eptaloprost, Ciprosten und/oder Beraprost und deren Salze zur Herstellung eines Arzneimittels zur Behandlung von chronischer Polyarthritis. Die Substanzen einschließlich Iloprost sind in der Tabelle 1 bezüglich ihrer Struktur aufgeführt.

Die Erfindung betrifft weiterhin die Verwendung von erfindungsgemäßen Prostan-Derivaten zusammen mit pharmakologischen Hilfs- und Trägerstoffen, die physiologisch verträglich sind. Solche Substanzen sind in Remington's Pharmaceutical Science, 15^{th} ed. Mack Publishing Company, Easton Pennsylvania (1980) beschrieben.

Die Herstellungsverfahren für einige der erfindungsgemäßen Prostan-Derivate sind ausführlich in der Publikation EP 0 011 591 oder EP 0 084 856 beschrieben. Die nicht ausdrücklich dort erwähnten Substanzen der Formel I oder II unterscheiden sich jedoch nicht im Bezug auf das Herstellungsverfahren. Sie sind vom Fachmann im Rahmen des Allgemeinwissens herstellbar.

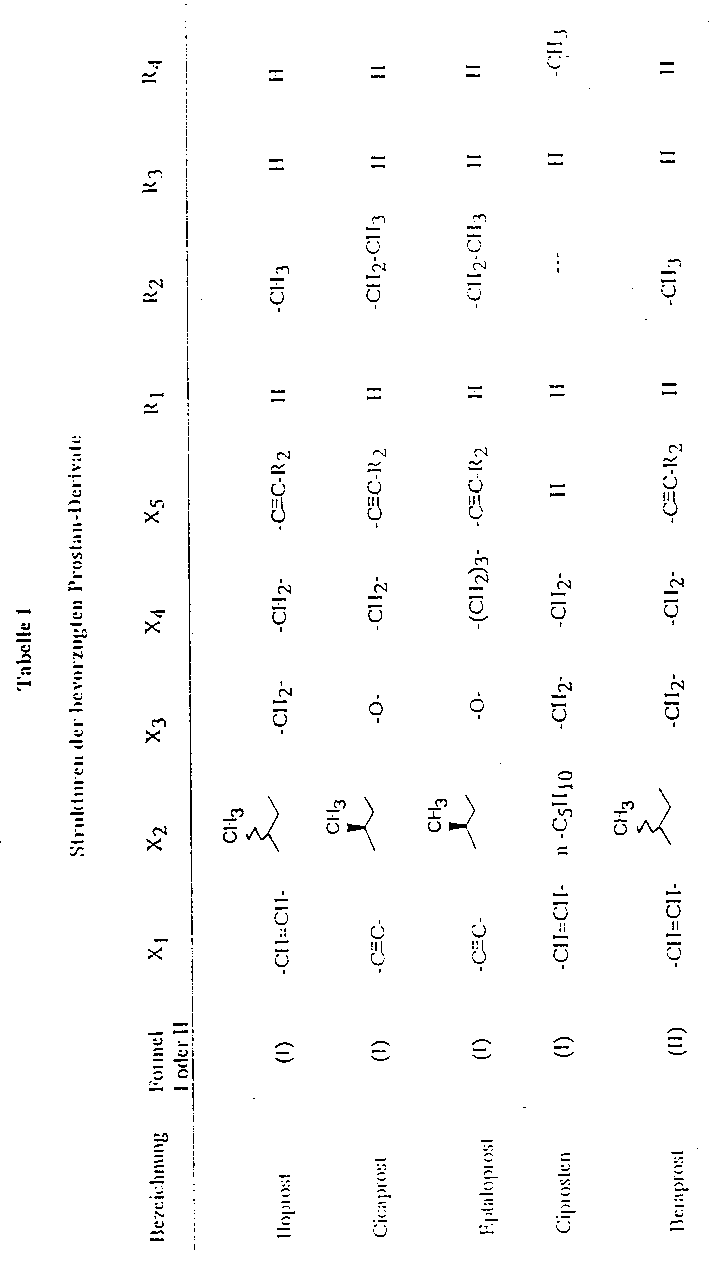

Die Verbindungen der allgemeinen Formel I oder II sind in dem nachfolgenden Test wirksam einsetzbar:

Zellen aus der Synovialflüssigkeit von Patienten mit chronischer Polyarthritis werden in vitro mit 4 Tage-alten Gliedknospen (Limbbuds) von Mäuse-Embryonen (12 Tage) kokultiviert. Nach einer Kokultivierung von ca. 7 Tagen findet ein Abbau des Knorpels in den Limbbuds statt, der in Anwesenheit von den erfindungsgemäßen Prostan-Derivaten reduziert werden kann.
Der Knorpelabbau wird über die Freisetzung von sulfatierten Glycosaminoglycanen gemessen, die als Alcian-Blau-Glycosaminoglycan-Komplex quantifiziert werden.
Die erfindungsgemäßen Prostan-Derivate reduzieren die Freisetzung der Glycosaminoglycanen, wobei die Freisetzung über die Synovialzellen induziert wird.
Elektronenmikroskopische Untersuchungen von Knorpel, der den aktivierten Synovialzellen von Patienten mit chronischer Polyarthritis ausgesetzt war, zeigt einen vollständigen Abbau an Kollagenfibrillen. In Anwesenheit von erfindungsgemäßen Prostan-Derivaten ist der Abbau an Kollagenfibrillen reduziert.

Das Testsystem zeigt biochemisch und elektronenmikroskopisch, daß der über Synovialzellen von Patienten mit chronischer Polyarthritis induzierte Knorpelabbau durch die erfindungsgemäßen Prostan-Derivate der Formel I oder II reduziert wird.

Die erfindungsgemäßen Prostan-Derivate zeigen die Wirkung in dem zuvor genannten Test bei Konzentrationen von 0,1 bis 1000 ng/ml. Das oben genannte *in vitro* Testsystem ist auf *in vivo* Prozesse übertragbar (vgl.: J.J. STEINBERG et al. (1983) Biochem Biophys Acta **757**: 47 - 58, weiterhin M.J. GROSSLEY and I.M. HUNNEYBALL (1984) Biochem Pharmac **33**: 1263 - 1271, weiterhin K. KELLER et al. (1990) J Orthop Res **8**: 345 - 352) Das *in vitro* System ist derart komplex, daß es repräsentativ für das Erkrankungsbild bei der chronischen Polyarthritis ist.

Die Verbindungen der allgemeinen Formel I oder II sind zur Behandlung von chronischer Polyarthritis geeignet.

Für diese therapeutische Wirkung ist die geeignete Dosis unterschiedlich und hängt beispielsweise von der verwendeten Verbindung der allgemeinen Formel I oder II, dem Wirt, der Art der Verabreichung und der Art und der Schwere der zu behandelnden Zustände ab. Im allgemeinen sind jedoch bei Tieren zufriedenstellende Resultate zu erwarten bei täglichen Dosen von 1 bis 3000 µg/kg Tierkörpergewicht. Bei größeren Säugetieren, beispielsweise Menschen, beträgt eine empfohlene tägliche Dosis 0,1 bis 200 mg der erfindungsgemäßen Prostan-Derivate der allgemeinen Formel I oder II. Bevorzugt sind Werte von 0,3 bis 60 mg pro Tag, mehr bevorzugt 1 bis 20 mg pro Tag und am meisten bevorzugt 2 bis 10 mg pro Tag. Die tägliche Dosis der Prostan-Derivate kann zweckmäßigerweise in 2 bis 4 Teildosen pro Tag verabreicht werden.

Die Prostan-Derivate der allgemeinen Formel I oder II können bei systemischer Behandlung auf jedem üblichen Weg verabreicht werden, insbesondere enteral, vorzugsweise oral, am meisten bevorzugt parenteral. Zäpfchen, Tabletten, Kapseln, Tropfen, Injektionslösungen oder Suspensionen sind die entsprechenden Formen für die Verabreichung.

Das Prostan-Derivat Iloprost ist die besonders bevorzugte Substanz. Sie wird beispielsweise bei größeren Säugetieren, z.B. Menschen, in der zuvor dargestellen Art verabreicht. Die Dosen sind dabei um den Faktor 2 kleiner, als zuvor angegeben wurde. Die Infusionslösung als Dauerinfusion in üblichen wäßrigen Lösungsmitteln, z.B. physiologischer Kochsalzlösung, ist die für die systemische Behandlung bevorzugte Verabreichungsform. Dabei werden 0,01 ng/kg/min bis 1,0 ng/kg/min verabreicht, bevorzugt 0,03 - 0,3 ng/kg/min, am meisten bevorzugt 0,1 ± 0,05 ng/kg/min.

Da die chronische Polyarthritis sich auch in schubweise verlaufenden Anfällen äußert, ist ein erfindungsgemäßes Prostan-Derivat auch während der symptomfreinen Zeit zu verabreichen. Somit dient das erfindungsgemäße Prostan-Derivat zur prophylaktischen Behandlung. Die Dosierungen bei der prophylaktischen Verabreichung weichen nicht von den zuvor beschriebenen wesentlich ab, jedoch sind Werte, die um den Faktor 2 bis 4 niedriger als die, die bei den akuten Schüben verwendet werden, bevorzugt.

Die vorliegende Erfindung betrifft ebenfalls therapeutische Zusammensetzungen, die ein Prostan-Derivat der allgemeinen Formel I oder II zusammen mit zumindest einem pharmazeutischen Träger, Zusatzstoff oder Verdünnungsmittel enthalten, die alle physiologisch verträglich sind. Solche Zusammensetzungen können auf an sich bekannte Weise hergestellt werden. Pharmakologisch verträgliche und geeignete Hilfs- und Trägerstoffe sind zum Beispiel in Remington's Pharmaceutical Science, 15^{th} ed. Mack Publishing Company, Easton Pennsylvania (1980) beschreiben. Ebenso ist die Verwendung von Cyclodextrin-Clathraten, die in der europäischen Patentschrift EP 0 259 468 aufgeführte sind, mögich.

Gegenüber der systemischen Behandlung ist die lokale Applikation mit einem erfindungsgemäßen Prostan-Derivat gegebenenfalls mit Hilf- und/oder Trägerstoffen besonders bevorzugt.

Bei höheren Konzentrationen von Prostan-Derivaten im Blut kommt die kardiovaskuläre Wirkung stark zum Tragen. Daher ist vorteilhaft, die erfindungsgemäßen Prostan-Derivate auf die Haut direkt am beeinträchtigten Gelenk aufzutragen. Dazu eignen sich Salben, Pasten, Gele oder Packungen. Diese Aufzählung ist nicht abschließend.

Durch die lokale Applikation wird in gewünschter Weise die Konzentration an Prostan-Derivaten in dem jeweiligen Gelenk auf einen hohen Wert angehoben, wobei gleichzeitig die Konzentration der Prostan-Derivate im Blut praktisch unter der Nachweisbarkeits-Grenze liegt. Die hohe Bioverfügbarkeit der Prostan-Derivate führt zu einer schnellen Durchdringung der Haut und des darunterliegenden Bindegewebes, wobei die Verteilung der Prostan-Derivate dem Diffusionsgesetz folgt. Dagegen spielt der Transport mittels Konvektionsströmung eine völlig unbedeutende Rolle. Die Gefäße, die in oder unter der Haut mit den aufgetragenen Prostan-Derivaten liegen, sind entweder zu klein (Venolen, Arteriolen) oder haben eine zu kleine effektive Oberfläche (Venen, Arterien) pro Hautpartie, um eine bemerkbare Konzentration an Prostan-Derivaten im Blut hervorzurufen.

### Beispiel Teil 1

### Zellkulturen aus der Synovialflüssgkeit

Zellen aus der Synovialflüssigkeit von Patienten mit chronischer Polyarthritis werden in vitro mit 4 Tage-alten Gliedknospen (Limbbuds) von Mäuse-Embryonen (12 Tage alt) kokultiviert. Nach einer Kokultivierung von ca. 7 Tagen findet ein Abbau des Knorpels in den Limbbuds statt, der in Anwesenheit von den erfindungsgemäßen Prostan-Derivaten reduziert werden kann.
Die Substanz Iloprost befindet sich in einer Endkonzentration von 100 ng/ml Kulturmedium in den Zellkulturen und wird beim Start zu den Zellkulturen hinzugegeben.
Proben werden an den Tagen 1 bis 7 genommen. Die Kulturbedingungen sind in H. MOHAMED-ALI (1991) Z Rheumatol **50**: 74 - 81; weiterhin in H.J. MERKER (1991) in Culture Techneques.D. NEUBERT and H.J. MERKER (eds) Walter de Gruyter Verlag, Berlin - New York, pp 119 - 133; weiterhin in H.B. FELL and R.W. JUBB (1977) Arthritis Rheuma **20**: 1359 - 1371 detailliert beschreiben.

Der Kontrollansatz enthält anstelle des Iloprosts lediglich Kulturmedium

### Beispiel Teil 2

### Messung des Knorpelabbaus

Der Knorpelabbau wird über die Freisetzung von sulfatierten Glycosaminoglycanen gemessen, die als Alcian-Blau-Glycosaminoglycan-Komplex quantifiziert werden. Die Freisetzung von Glycosaminoglycan wird in P. WHITEMAN (1973) Biochem J **131**: 343 - 351 beschrieben. Die an den Tagen 1 bis 7 genommenen Proben zeigen die in Tabelle 2 aufgelisteten Ergebnisse.

**Tabelle 2**

| Tag der Probenahme: | 1 | 3 | 4 | 5 | 6 | 7 |
|---|---|---|---|---|---|---|
| Kultur mit Iloprost ¹⁾ | 7 | 13 | 14 | 12 | 11 | 13 |
| Kontrolle ¹⁾ | 11 | 24 | 19 | 22 | 21 | 21 |

| | | | | | | |
|---|---|---|---|---|---|---|
| ¹⁾ = Werte in µg Glucosaminglucan pro ml Medium | | | | | | |

Die erfindungsgemäßen Prostan-Derivate reduzieren die Freisetzung der Glycosaminoglycanen signifikant.

### Beispiel Teil 3

### Elektronenmikroskopische Auswertung

Elektronenmikroskopische Untersuchungen von Knorpel, der den aktivierten Synovialzellen von Patienten mit chronischer Polyarthritis ausgesetzt war, zeigt einen vollständigen Abbau an Kollagenfibrillen. In Anwesenheit von erfindungsgemäßen Prostan-Derivaten ist der Abbau an Kollagenfibrillen reduziert.
Die Herstellung der elektronenmikroskopischen Präparate und die histologische Auswertung ist in H. MOHAMED-ALI (1991) Z Rheumatol **50**: 74 - 81; weiterhin in H.J. MERKER (1991) in Culture Techneques.D. NEUBERT and H.J. MERKER (eds) Walter de Gruyter Verlag, Berlin - New York, pp 119 - 133; weiterhin in H.B. FELL and R.W. JUBB (1977) Arthritis Rheuma **20**: 1359 - 1371 beschrieben. Die mit dem erfindungsgemäßen Prostan-Derivat behandleten Kulturzellen aus Patienten mit chronischer Polyarthritis liegen in ihrem Erscheinungsbild zwischen Zellen aus Patienten mit chronischer Polyarthritis ohne Prostan-Derivat-Behandlung und Zellen aus Patienten ohne chronischer Polyarthritis. Die Reduktion des Kollagen-Abbaues wird aufgrund der Präparate auf 50 % eingeschätzt

## Patentansprüche

1. Verwendung von Prostan-Derivaten der allgemeinen Formel I oder II 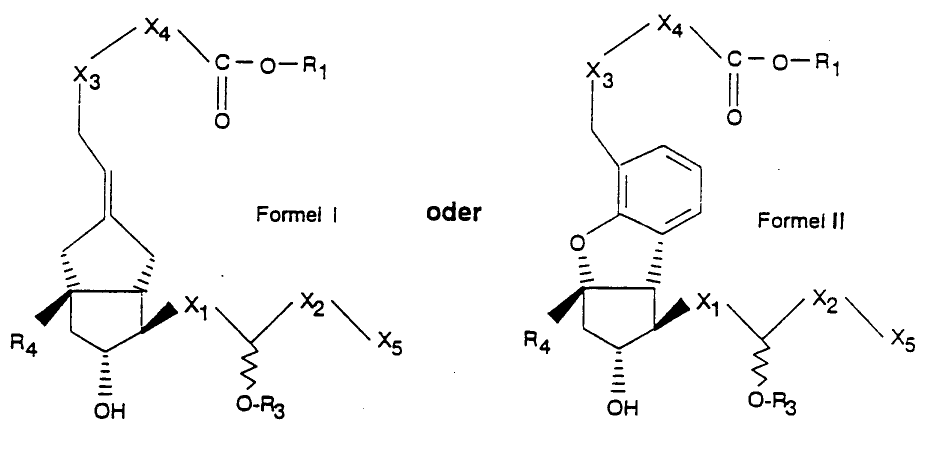 worin
X₁ ein -CH₂-CH₂-; trans -CH=CH- oder ein -C≡C- ist ist,
X₂ eine geradkettige oder verzweigte gesättigte Alkylengruppe mit 1 bis 6 Kohlenstoff-Atomen ist,
X₃ ein -O oder -CH₂- ist,
X₄ ein -CH₂- oder ein -[CH₂]₃- ist,
X₅ ein -H oder ein -C≡C-R₂ ist;
R₁ ein Wasserstoff-Atom, eine Alkylgruppe mit 1 bis 6 Kohlenstoff-Atomen, eine Cycloalkylgruppe mit 5 oder 6 Kohlenstoff-Atomen oder Phenylgruppe ist,
R₂ eine gerad- oder verzweigtkettige, gesättigte oder ungesättigte Alkylgruppe mit 1 bis 6 Kohlenstoff-Atomen ist,
R₃ ein Wasserstoff-Atom, ein Acylrest mit 1 bis 4 Kohlenstoff-Atomen oder eine Benzoylrest ist und
R₄ ein -H oder -CH₃ ist;
wobei die -O-R₃-Gruppe α- oder β- ständig ist,
und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

2. Verwendung von Prostan-Derivaten nach Anspruch 1 mit der allgemeinen Formel I
worin
X₁ ein trans -CH=CH- ist,
X₂ eine geradkettige oder verzweigte gesättigte Alkylengruppe mit 2 bis 4 Kohlenstoff-Atomen ist,
X₃ ein -CH₂- ist,
X₄ ein -CH₂- ist,
X₅ ein -C≡C-R₂ ist;
R₁ ein Wasserstoff-Atom, ein Alkylgruppe mit 1 bis 3 Kohlenstoff-Atomen oder Phenylgruppe ist,
R₂ eine gerad- oder verzweigtkettige, gesättigte oder ungesättigte Alkylgruppe mit 1 bis 3 Kohlenstoff-Atomen ist,
R₃ ein Wasserstoff-Atom oder ein Acylrest mit 2 Kohlenstoff-Atomen ist und
R₄ ein -H ist;
wobei die -O-R₃-Gruppe α- oder β- ständig ist, und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

3. Verwendung von Prostan-Derivaten nach Anspruch 2 mit der allgemeinen Formel I
worin
X₁ ein trans -CH=CH- ist,
X₂ eine Methylethylengruppe ist, wobei die Methylgruppe mit dem ersten Kohlenstoff-Atom der Ethylengruppe verbunden ist und das erste Kohlenstoff-Atom der Ethylengruppe zu der Gruppe A weist,
X₃ ein -CH₂- ist,
X₄ ein -CH₂- ist,
X₅ ein -C≡C-R₂ ist;
R₁ ein Wasserstoff-Atom oder eine Methylgruppe ist,
R₂ eine Methyl- oder Ethylgruppe ist,
R₃ ein Wasserstoff-Atom oder ein Formylrest ist und
R₄ ein -H ist;
wobei die -O-R₃-Gruppe α- oder β- ständig ist,
und deren Salze mit physiologisch verträglichen Basen, falls R₁ die Bedeutung eines Wasserstoff-Atoms hat,
zur Herstellung eines Arzneimittels zur Behandlung von chronischer Polyarthritis.

4. Verwendung von einem Prostan-Derivat nach Anspruch 3 mit der Formel I
worin
X₁ trans -CH=CH- ist,
X₂ eine Methylethylengruppe ist, wobei die Methylgruppe mit dem ersten Kohlenstoff-Atom der Ethylengruppe verbunden ist und das erste Kohlenstoff-Atom der Ethylengruppe zu der Gruppe A weist,
X₃ ein -CH₂- ist,
X₄ ein -CH₂- ist,
X₅ ein -C≡C-R₂ ist;
R₁ ein Wasserstoff-Atom ist,
R₂ eine Methlygruppe ist,
R₃ ein Wasserstoff-Atom ist und
R₄ ein -H ist;
wobei die OH-Gruppe α- oder β- ständig ist,
und dessen Salze mit physiologisch verträglichen Basen zur Herstellung eines Arzneimittels zur Behandlung von chronischer Polyarthritis.

5. Verwendung von einem Prostan-Derivat nach Anspruch 1, das ein Cicaprost ist, und dessen Salze
zur Herstellung eines Arzneimittels zur Behandlung von chronischer Polyarthritis.

6. Verwendung von einem Prostan-Derivat nach Anspruch 1, das ein Eptaloprost ist, und dessen Salze
zur Herstellung eines Arzneimittels zur Behandlung von chronischer Polyarthritis.

7. Verwendung von einem Prostan-Derivat nach Anspruch 1, das ein Ciprosten ist, und dessen Salze
zur Herstellung eines Arzneimittels zur Behandlung von chronischer Polyarthritis.

8. Verwendung von einem Prostan-Derivat nach Anspruch 1, das ein Beraprost ist, und dessen Salze
zur Herstellung eines Arzneimittels zur Behandlung von chronischer Polyarthritis.

9. Verwendung von Prostan-Derivaten nach wenigstens einem der vorherigen Ansprüche zusammen mit pharmakologischen Hilfs- und Trägerstoffen, die physiologisch verträglich sind.

## Claims

1. Use of prostane derivatives of general formula I or II 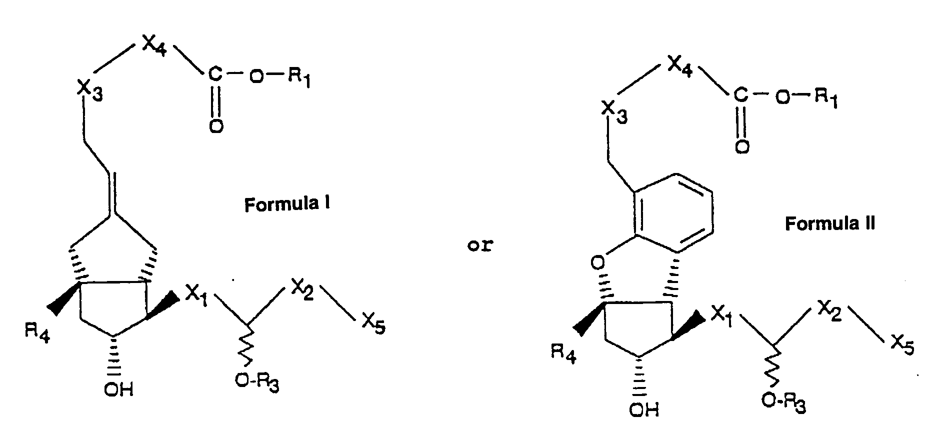 in which
X₁ is a -CH₂-CH₂-; trans -CH=CH- or a -C≡C-,
X₂ is a straight-chain or branched, saturated alkylene group with 1 to 6 carbon atoms,
X₃ is an -O- or -CH₂-,
X₄ is a -CH₂- or a -[CH₂]₃-,
X₅ is an -H or a -C≡C-R₂;
R₁ is a hydrogen atom, an alkyl group with 1 to 6 carbon atoms, a cycloalkyl group with 5 or 6 carbon atoms, or a phenyl group,
R₂ is a straight-chain or branched-chain, saturated or unsaturated alkyl group with 1 to 6 carbon atoms,
R₃ is a hydrogen atom, an acyl radical with 1 to 4 carbon atoms, or a benzoyl radical, and
R₄ is an -H or -CH₃;
in which the -O-R₃ group is in α- or β-position, and their salts with physiologically compatible bases, if R₁ indicates a hydrogen atom,
for the production of a pharmaceutical agent for the treatment of chronic polyarthritis.

2. Use of prostane derivatives according to claim 1 with general formula I
in which
X₁ is a trans -CH=CH-,
X₂ is a straight-chain or branched, saturated alkylene group with 2 to 4 carbon atoms,
X₃ is a -CH₂-,
X₄ is a -CH₂-,
X₅ is a -C≡C-R₂;
R₁ is a hydrogen atom, an alkyl group with 1 to 3 carbon atoms, or a phenyl group,
R₂ is a straight-chain or branched-chain, saturated or unsaturated alkyl group with 1 to 3 carbon atoms,
R₃ is a hydrogen atom or an acyl radical with 2 carbon atoms and
R₄ is an -H;
in which the -O-R₃ group is in α- or β-position, and their salts with physiologically compatible bases, if R₁ indicates a hydrogen atom,
for the production of a pharmaceutical agent for the treatment of chronic polyarthritis.

3. Use of prostane derivatives according to claim 2 with general formula I
in which
X₁ is a trans -CH=CH-,
X₂ is a methylethylene group in which the methyl group is connected to the first carbon atom of the ethylene group and the first carbon atom of the ethylene group is assigned to group A,
X₃ is a -CH₂-,
X₄ is a -CH₂-,
X₅ is a -C≡C-R₂;
R₁ is a hydrogen atom or a methyl group,
R₂ is a methyl or ethyl group,
R₃ is a hydrogen atom or a formyl group, and
R₄ is an -H;
in which the -O-R₃ group is in α- or β-position and their salts with physiologically compatible bases, if R₁ indicates a hydrogen atom,
for the production of a pharmaceutical agent for the treatment of chronic polyarthritis.

4. Use of a prostane derivative according to claim 3 with formula I
in which
X₁ is a trans -CH=CH-,
X₂ is a methylethylene group, in which the methyl group is connected to the first carbon atom of the ethylene group and the first carbon atom of the ethylene group is assigned to group A,
X₃ is a -CH₂-,
X₄ is a -CH₂-,
X₅ is a -C≡C-R₂;
R₁ is a hydrogen atom,
R₂ is a methyl group,
R₃ is a hydrogen atom, and
R₄ is an -H;
in which the OH group is in α- or β-position, and its salts with physiologically compatible bases,
for the production of a pharmaceutical agent for the treatment of chronic polyarthritis.

5. Use of a prostane derivative according to claim 1, which is a cicaprost, and its salts
for the production of a pharmaceutical agent for the treatment of chronic polyarthritis.

6. Use of a prostane derivative according to claim 1, which is an eptaloprost, and its salts
for the production of a pharmaceutical agent for the treatment of chronic polyarthritis.

7. Use of a prostane derivative according to claim 1, which is a ciprostene, and its salts
for the production of a pharmaceutical agent for the treatment of chronic polyarthritis.

8. Use of a prostane derivative according to claim 1, which is a beraprost, and its salts
for the production of a pharmaceutical agent for the treatment of chronic polyarthritis.

9. Use of prostane derivatives according to at least one of the prior claims together with pharmacological adjuvants and vehicles, which are physiologically compatible.

## Revendications

1. Utilisation de dérivés du prostane de formule générale I ou II 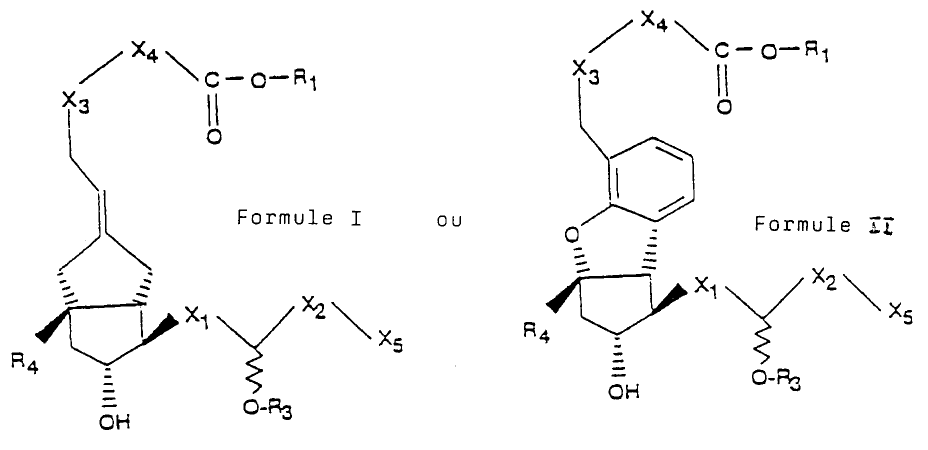 dans lesquelles
X₁ est un -CH₂-CH₂-; un -CH=CH- trans; ou un -C≡C-,
X₂ est un groupe alkylène saturé, rectiligne ou ramifié, avec 1 à 6 atomes de carbone,
X₃ est un -O- ou un -CH₂-,
X₄ est un -CH₂- ou un -[CH₂]₃-,
X₅ est un -H ou un -C≡C-R₂;
R₁ est un atome d'hydrogène, un groupe alkyle avec 1 à 6 atomes de carbone, un groupe cycloalkyle avec 5 ou 6 atomes de carbone ou le groupe phényle,
R₂ est un groupe alkyle, saturé ou insaturé, rectiligne ou ramifié, avec 1 à 6 atomes de carbone,
R₃ est un atome d'hydrogène, un radical acyle avec 1 à 4 atomes de carbone ou un radical benzoyle et
R₄ est un -H ou -CH₃;
le groupe -O-R₃ étant en position α ou β,
et de leurs sels avec des bases physiologiquement supportables, dans le cas où R₁ a la signification d'un atome d'hydrogène,
pour la préparation d'un médicament pour le traitement de la polyarthrite chronique.

2. Utilisation de dérivés du prostane selon la revendication 1, ayant la formule générale I
dans laquelle
X₁ est un -CH=CH- trans,
X₂ est un groupe alkylène saturé, rectiligne ou ramifié, avec 2 à 4 atomes de carbone,
X₃ est un -CH₂-,
X₄ est un -CH₂-,
X₅ est un -C≡C-R₂,
R₁ est un atome d'hydrogène, un groupe alkyle avec 1 à 3 atomes de carbone ou le groupe phényle,
R₂ est un groupe alkyle saturé ou insaturé, rectiligne ou ramifié, avec 1 à 3 atomes de carbone,
R₃ est un atome d'hydrogène ou un radical acyle avec 2 atomes de carbone et
R₄ est un -H;
le groupe -O-R₃ étant en position α ou β,
et de leurs sels avec des bases physiologiquement supportables, dans le cas où R₁ a la signification d'un atome d'hydrogène,
pour la préparation d'un médicament pour le traitement de la polyarthrite chronique.

3. Utilisation de dérivés du prostane selon la revendication 2, ayant la formule générale I
dans laquelle
X₁ est un -CH=CH- trans,
X₂ est un groupe méthyléthylène, le groupe méthyle étant lié au premier atome de carbone du groupe éthylène et le premier atome de carbone du groupe éthylène attribuant le groupe A,
X₃ est un -CH₂-,
X₄ est un -CH₂-,
X₅ est un -C≡C-R₂;
R₁ est un atome d'hydrogène ou un groupe méthyle,
R₂ est un groupe méthyle ou éthyle,
R₃ est un atome d'hydrogène ou un radical formyle et
R₄ est un -H;
le groupe -O-R₃ étant en position α ou β,
et de leurs sels avec des bases physiologiquement supportables, dans le cas où R₁ a la signification d'un atome d'hydrogène, pour la préparation d'un médicament pour le traitement de la polyarthrite chronique.

4. Utilisation d'un dérivé du prostane selon la revendication 3, ayant la formule générale I
dans laquelle
X₁ est un -CH=CH- trans,
X₂ est un groupe méthyléthylène, le groupe méthyle étant lié au premier atome de carbone du groupe éthylène et le premier atome de carbone du groupe éthylène attribuant le groupe A,
X₃ est un -CH₂-,
X₄ est un -CH₂-,
X₅ est un -C≡C-R₂;
R₁ est un atome d'hydrogène,
R₂ est un groupe méthyle,
R₃ est un atome d'hydrogène et
R₄ est un -H;
le groupe OH étant en position α ou β,
et de ses sels avec des bases physiologiquement supportables,
pour la préparation d'un médicament pour le traitement de la polyarthrite chronique.

5. Utilisation d'un dérivé du prostane selon la revendication 1, qui est un cicaprost, et de ses sels
pour la préparation d'un médicament pour le traitement de la polyarthrite chronique.

6. Utilisation d'un dérivé du prostane selon la revendication 1, qui est un eptaloprost, et de ses sels
pour la préparation d'un médicament pour le traitement de la polyarthrite chronique.

7. Utilisation d'un dérivé du prostane selon la revendication 1, qui est un ciprostène, et de ses sels
pour la préparation d'un médicament pour le traitement de la polyarthrite chronique.

8. Utilisation d'un dérivé du prostane selon la revendication 1, qui est un beraprost, et de ses sels
pour la préparation d'un médicament pour le traitement de la polyarthrite chronique.

9. Utilisation de dérivés du prostane selon au moins une des revendications précédentes, ensemble avec des adjuvants et excipients pharmacologiques qui sont physiologiquement supportables.
